# EUROPEAN PATENT APPLICATION

(11) **EP 3 403 607 A1**
(43) Date of publication of application: **21.11.2018**
(21) Application number: 18172047.5
(22) Date of filing: 14.05.2018
(51) Int. Cl.: A61B 18/14

(54) **NETWORKED THERMISTORS**

(30) Priority: 15.05.2017 US 201715594866
(71) Applicant: Biosense Webster (Israel) Ltd., Yokneam 2066717 (IL)
(72) Inventor: BAR-TAL, Meir, 2066717 Yokneam (IL); OSADCHY, Daniel, 2066717 Yokneam (IL)
(74) Representative: Small, Gary James

(57) **Abstract**

A medical catheter has an inflatable balloon assembly on its distal portion and a plurality of ablation electrodes disposed on the surface of the balloon assembly. Conductors extending through the shaft supply the ablation electrodes with electrical power when connected to a power source. A plurality of thermistors on the balloon assembly are connected to the conductors to form a resistive network, and a plurality of access points on the conductors are provided for obtaining electrical measurements and analyzing the measurements to determine respective resistances of the thermistors.

## Description

### COPYRIGHT NOTICE

A portion of the disclosure of this patent document contains material that is subject to copyright protection. The copyright owner has no objection to the facsimile reproduction by anyone of the patent document or the patent disclosure, as it appears in the Patent and Trademark Office patent file or records, but otherwise reserves all copyright rights whatsoever.

### BACKGROUND OF THE INVENTION

### 1. Field of the Invention.

This invention relates to invasive medical devices. More particularly, this invention relates to detecting the temperature of the interior portion of the body.

### 2. Description of the Related Art.

Ablation of body tissue using electrical energy is known in the art. The ablation is typically performed by applying alternating currents, for example radiofrequency energy, to the electrodes, at a sufficient power to destroy target tissue. Typically, the electrodes are mounted on the distal tip of a catheter, which is inserted into a subject. The distal tip may be tracked in a number of different ways known in the art, for example by measuring magnetic fields generated at the distal tip by coils external to the subject.

A known difficulty in the use of radiofrequency energy for cardiac tissue ablation is controlling local heating of tissue. There are tradeoffs between the 15 desire to create a sufficiently large lesion to effectively ablate an abnormal tissue focus, or block an aberrant conduction pattern, and the undesirable effects of excessive local heating. If the radiofrequency device creates too small a lesion, then the medical procedure could be less effective, or could require too much time. On the other hand, if tissues are heated excessively then there could be local charring 20 effects due to overheating. Such overheated areas can develop high impedance, and may form a functional barrier to the passage of heat. The use of slower heating provides better control of the ablation, but unduly prolongs the procedure.

For a balloon catheter used for ablation, it is important to measure the temperature of the tissue in contact with the balloon. In one approach, U.S. Patent No. 7,081,096 proposes a device for locating inflammation and increased metabolic activity associated with conditions such as vulnerable plaque by mapping temperature of a body lumen, such as an artery or blood vessel. The temperature mapping balloon comprises a balloon with a thermal mapping coating disposed on the inside or outside of the balloon. The thermal mapping coating can be a thermochromic coating that changes color in response to temperature or a sensor coating comprising a plurality of temperature sensors. The temperatures are read by deflating and withdrawing the balloon, then reading temperature maps using optical scanning machines.

In a balloon catheter adding temperature sensors such as thermistors or thermocouples normally requires adding extra leads to each sensor. This is problematic because of the limited amount of "real estate" available for the extra leads.

### SUMMARY OF THE INVENTION

According to disclosed embodiments of the invention, thermistors are used as the temperature sensors in a balloon ablation catheter, but rather than connecting separate leads to the thermistors, the leads to the ablating electrodes are also used for the thermistors.

There is provided according to embodiments of the invention a medical catheter having an inflatable balloon assembly on its distal portion and a plurality of ablation electrodes disposed on the surface of the balloon assembly. Conductors extending through the shaft supply the ablation electrodes with electrical power when connected to a power source. A plurality of thermistors on the balloon assembly are connected to the conductors to form a resistive network, and a plurality of access points on the conductors are provided for obtaining electrical measurements therefrom.

According to one aspect of the apparatus, the network has nodes connected to respective voltage or current sources.

According to a further aspect of the apparatus, the respective voltage or current sources have respective frequencies.

According to yet another aspect of the apparatus, the balloon assembly has a plurality of splines, and the ablation electrodes and the thermistors are electrically connected with the splines.

According to still another aspect of the apparatus, the splines comprise flexible circuitry, and the thermistors are mounted on the splines.

According to another aspect of the apparatus, there are eight access points and the splines each have four thermistors mounted thereon.

According to an additional aspect of the apparatus, the splines are connected by bridging connectors to define electromagnetic loops.

According to one aspect of the apparatus, there are four splines sharing a common distal electrical connection.

According to a further aspect of the apparatus, the ablation electrodes are placed on the surface of the balloon assembly by printing.

According to yet another aspect of the apparatus, the ablation electrodes are placed on the surface of the balloon assembly by stamping.

There is further provided according to embodiments of the invention a method which is carried out by inserting a catheter into the body of a subject. The distal portion of the catheter has an inflatable balloon assembly and a plurality of ablation electrodes on the surface of the balloon assembly. The method is further carried out by connecting the ablation electrodes to a power source via conductors extending through the shaft to supply the ablation electrodes with electrical power. A plurality of thermistors are disposed on the balloon assembly in electrical connection with the conductors to form a resistive network. The method is further carried out by obtaining electrical measurements at a plurality of access points on the conductors, and analyzing the electrical measurements to derive respective resistances of the thermistors.

### BRIEF DESCRIPTION OF THE SEVERAL VIEWS OF THE DRAWINGS

For a better understanding of the present invention, reference is made to the detailed description of the invention, by way of example, which is to be read in conjunction with the following drawings, wherein like elements are given like reference numerals, and wherein:
Fig. 1 is a pictorial illustration of a system for performing catheterization procedures on a heart, in accordance with a disclosed embodiment of the invention;
Fig. 2 is a partially schematic view of an expanded balloon catheter in accordance with an embodiment of the invention;
Fig. 3 is a schematic diagram of a resistive network defined by conductors in the embodiment of Fig. 2 in accordance with an embodiment of the invention;
Fig. 4 is an elevation of the distal portion of an expanded cardiac balloon catheter in accordance with an alternate embodiment of the invention;
Fig. 5 is an elevation of the distal portion of an expanded cardiac balloon catheter in accordance with an alternate embodiment of the invention; and
Fig. 6 is a partially schematic diagram of the distal portion of an expanded cardiac balloon catheter in accordance with an alternate embodiment of the invention.

### DETAILED DESCRIPTION OF THE INVENTION

In the following description, numerous specific details are set forth in order to provide a thorough understanding of the various principles of the present invention. It will be apparent to one skilled in the art, however, that not all these details are necessarily needed for practicing the present invention. In this instance, well-known circuits, control logic, and the details of computer program instructions for conventional algorithms and processes have not been shown in detail in order not to obscure the general concepts unnecessarily.

Documents incorporated by reference herein are to be considered an integral part of the application except that, to the extent that any terms are defined in these incorporated documents in a manner that conflicts with definitions made explicitly or implicitly in the present specification, only the definitions in the present specification should be considered.

### System Overview.

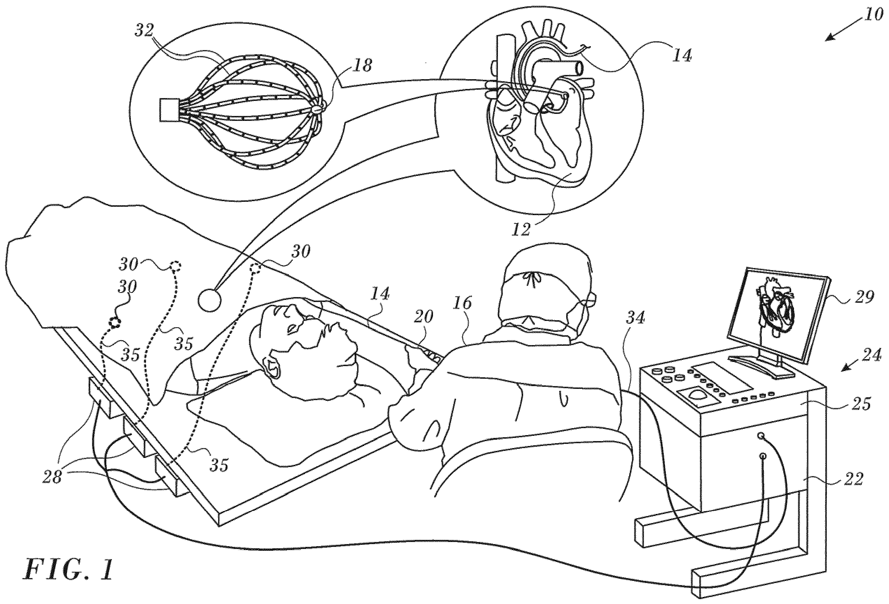

Turning now to the drawings, reference is initially made to Fig. 1, which is a pictorial illustration of a system 10 for evaluating electrical activity and performing ablative procedures on a heart 12 of a living subject, which is constructed and operative in accordance with a disclosed embodiment of the invention. The system comprises a catheter 14, which is percutaneously inserted by an operator 16 through the patient's vascular system into a chamber or vascular structure of the heart 12. The operator 16, who is typically a physician, brings the catheter's distal tip 18 into contact with the heart wall, for example, at an ablation target site. Electrical activation maps may be prepared, according to the methods disclosed in U.S. Patent Nos. 6,226,542, and 6,301,496, and in commonly assigned U.S. Patent No. 6,892,091, whose disclosures are herein incorporated by reference. One commercial product embodying elements of the system 10 is available as the CARTO® 3 System, available from Biosense Webster, Inc., 3333 Diamond Canyon Road, Diamond Bar, CA 91765. This system may be modified by those skilled in the art to embody the principles of the invention described herein.

Areas determined to be abnormal, for example by evaluation of the electrical activation maps, can be ablated by application of thermal energy, e.g., by passage of radiofrequency electrical current through wires in the catheter to one or more electrodes at the distal tip 18, which apply the radiofrequency energy to the myocardium. The energy is absorbed in the tissue, heating it to a point (typically about 60°C) at which it permanently loses its electrical excitability. When successful, this procedure creates non-conducting lesions in the cardiac tissue, which disrupt the abnormal electrical pathway causing the arrhythmia. The principles of the invention can be applied to different heart chambers to diagnose and treat many different cardiac arrhythmias.

The catheter 14 typically comprises a handle 20, having suitable controls on the handle to enable the operator 16 to steer, position and orient the distal end of the catheter as desired for the ablation. To aid the operator 16, the distal portion of the catheter 14 contains position sensors (not shown) that provide signals to a processor 22, located in a console 24. The processor 22 may fulfill several processing functions as described below.

Ablation energy and electrical signals can be conveyed to and from the heart 12 through one or more ablation electrodes 32 located at or near the distal tip 18 via cable 34 to the console 24. Pacing signals and other control signals may be conveyed from the console 24 through the cable 34 and the electrodes 32 to the heart 12. Sensing electrodes 33, also connected to the console 24 are disposed between the ablation electrodes 32 and have connections to the cable 34.

Wire connections 35 link the console 24 with body surface electrodes 30 and other components of a positioning sub-system for measuring location and orientation coordinates of the catheter 14. The processor 22 or another processor (not shown) may be an element of the positioning subsystem. The electrodes 32 and the body surface electrodes 30 may be used to measure tissue impedance at the ablation site as taught in U.S. Patent No. 7,536,218, issued to Govari et al.*,* which is herein incorporated by reference. A temperature sensor (not shown), typically a thermocouple or thermistor, may be mounted on or near each of the electrodes 32.

The console 24 typically contains one or more ablation power generators 25. The catheter 14 may be adapted to conduct ablative energy to the heart using any known ablation technique, e.g., radiofrequency energy, ultrasound energy, and laser-produced light energy. Such methods are disclosed in commonly assigned U.S. Patent Nos. 6,814,733, 6,997,924, and 7,156,816, which are herein incorporated by reference.

In one embodiment, the positioning subsystem comprises a magnetic position tracking arrangement that determines the position and orientation of the catheter 14 by generating magnetic fields in a predefined working volume and sensing these fields at the catheter, using field generating coils 28. The positioning subsystem is described in U.S. Patent No. 7,756,576, which is hereby incorporated by reference, and in the above-noted U.S. Patent No. 7,536,218.

As noted above, the catheter 14 is coupled to the console 24, which enables the operator 16 to observe and regulate the functions of the catheter 14. Console 24 includes a processor, preferably a computer with appropriate signal processing circuits. The processor is coupled to drive a monitor 29. The signal processing circuits typically receive, amplify, filter and digitize signals from the catheter 14, including signals generated by sensors such as electrical, temperature and contact force sensors, and a plurality of location sensing electrodes (not shown) located distally in the catheter 14. The digitized signals are received and used by the console 24 and the positioning system to compute the position and orientation of the catheter 14, and to analyze the electrical signals from the electrodes.

In order to generate electroanatomic maps, the processor 22 typically comprises an electroanatomic map generator, an image registration program, an image or data analysis program and a graphical user interface configured to present graphical information on the monitor 29.

Typically, the system 10 includes other elements, which are not shown in the figures for the sake of simplicity. For example, the system 10 may include an electrocardiogram (ECG) monitor, coupled to receive signals from one or more body surface electrodes, in order to provide an ECG synchronization signal to the console 24. As mentioned above, the system 10 typically also includes a reference position sensor, either on an externally-applied reference patch attached to the exterior of the subject's body, or on an internally-placed catheter, which is inserted into the heart 12 maintained in a fixed position relative to the heart 12. Conventional pumps and lines for circulating liquids through the catheter 14 for cooling the ablation site are provided. The system 10 may receive image data from an external imaging modality, such as an MRI unit or the like and includes image processors that can be incorporated in or invoked by the processor 22 for generating and displaying images.

### First Alternate Embodiment.

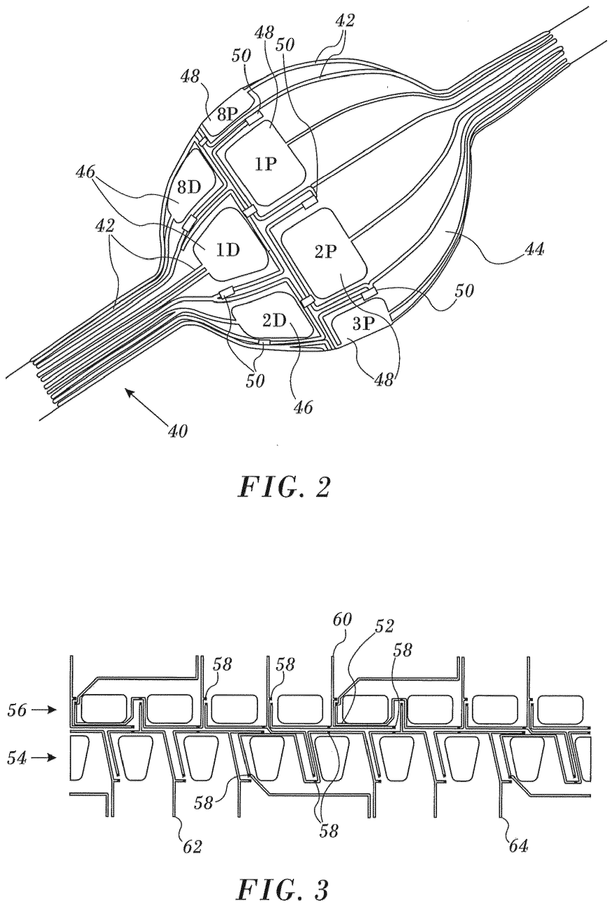

Reference is now made to Fig. 2, which is a partially schematic view of the distal portion of an expanded balloon catheter 40 in accordance with an embodiment of the invention. A network of leads 42 extending from the proximal end (not shown) of the catheter 40 supply multiple ablation electrodes, which are disposed on the surface of a balloon 44. In the configuration shown in Fig. 2, the leads 42 supplies proximal and distal groups of electrodes 46, 48, respectively. This number and configuration of ablation electrodes is exemplary, and the principles of the invention are applicable to many configurations and any number of ablation electrodes. Moreover, the network of leads may vary and assume many configurations. For example, there may be one subnetwork that supplies the proximal group of electrodes 46 and another that supplies the distal group of electrodes 48. The subnetworks may be independent or interconnected.

Included in the leads 42 are multiple thermistors 50, which are positioned generally near the electrodes 46, 48 in order that they contact the endocardial surface at or near the ablation site and that the readings accurately reflect the temperatures at the ablation site. In Fig. 2 there are a total of 24 thermistors.

Reference is now made to Fig. 3, which is a schematic diagram of the arrangement of a resistive network defined by leads 42 (Fig. 2) in accordance with an embodiment of the invention. The leads 42 are represented by lines 52. Rows 54, 56 represent the proximal and distal electrodes 46, 48, respectively. Thermistors 50 are not shown explicitly, but are realized as connections between pairs of dots 58. The proximity of the pairs of dots 58 to the electrodes 46, 48 is represented. Connections between the electrodes and the lines 52 corresponding to the leads 42 (Fig. 2) are not shown explicitly but will be understood.

In order to determine the temperatures represented by the resistances of individual thermistors 50, Voltage and current readings are taken at external measuring points of the network formed by the lines 52, e.g., points 60, 62, 64. The number of measuring points is at least sufficient to derive the resistances of the individual thermistors 50 by applying well-known principles of linear network analysis to the topology of the particular network formed by the lines 52 and solving the resulting linear system of equations. The network topology may vary according to the number and positions of the ablation electrodes on the balloon; nevertheless the principles are applicable by choosing suitable external measuring points to derive the resistance of each thermistor, using logical circuitry or a processor in the console 24 (Fig. 1). To implement the voltage and current measurement a source of voltage (or current) is implemented at each node of the network. The frequency of the sources are selected in a way that they can be detected independently of any other signal that may be present during the measurement (e.g., ablation signal, magnetic tracker signal or ECG signal), thus allowing a continuous resistance measurement. For example each node may be connected to a source having a respective frequency.

### Second Alternate Embodiment.

The thermistors may be incorporated into the leads supplying the ablation electrodes in different ways, for example, by stamping or printing, or combinations thereof, as known in the art. Flexible circuitry may be employed.

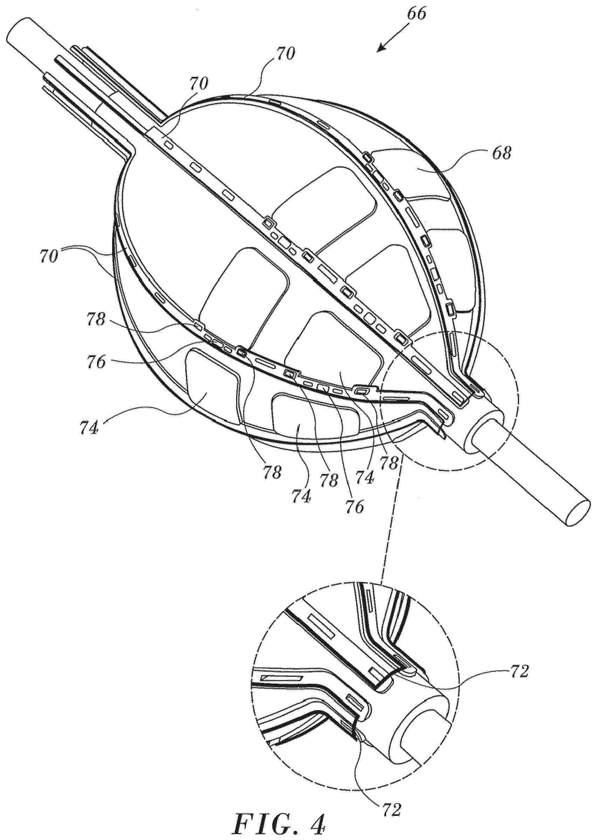

Reference is now made to Fig. 4, which is an elevation of the distal portion of an expanded cardiac balloon catheter 66 in accordance with an alternate embodiment of the invention. Balloon 68 has eight splines 70, of which five are visible. Distal bridging connections 72 join pairs of the splines 70 to define electromagnetic loops. Ablation electrodes 74 have connections 76 with the splines 70. The electrodes 74 are located on the surface of the balloon 68 and are positioned near thermistors 78. Typically there are four thermistors per spline, which are mounted on flexible circuitry that forms the splines. The thermistors 78 share the conductors leading to the electrodes 74. The resistances of the thermistors 78 are measured by taking voltage and current readings at eight access points along the proximal continuations (not shown) of the splines 70. In general the interconnections among the lines become more complex as the number of thermistors increases and the number of access points required to analyze the resistive network increases as well.

### Third Alternate Embodiment.

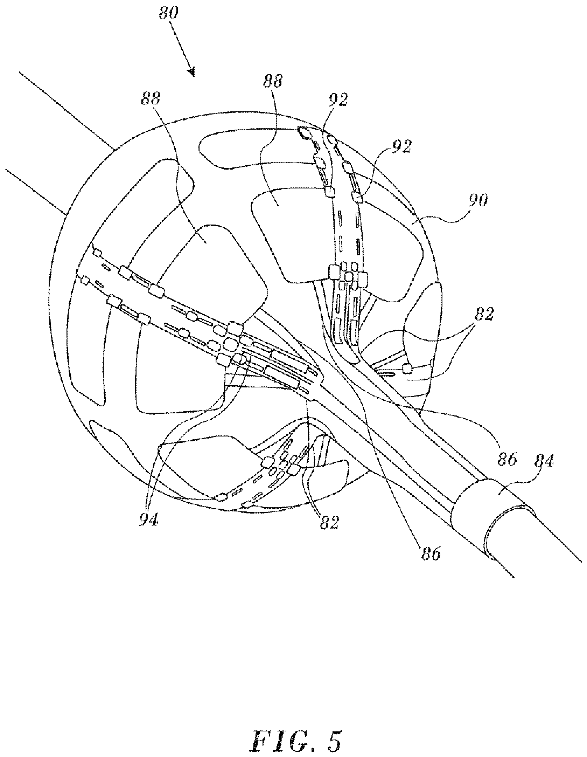

Reference is now made to Fig. 5, which is an elevation of the distal portion of an expanded cardiac balloon catheter 80 in accordance with an alternate embodiment of the invention. In this version there are four splines 82 having a common distal connection 84 and individual connections 86 to ablation electrodes 88, which are applied to the surface of balloon 90, e.g., by printing or stamping. Two rows of thermistors 92 appear on each of the splines 82 (typically four thermistors per spline), and are connected in a network by flexible circuit conductors, e.g., leads 94.

### Electromagnetic Loops.

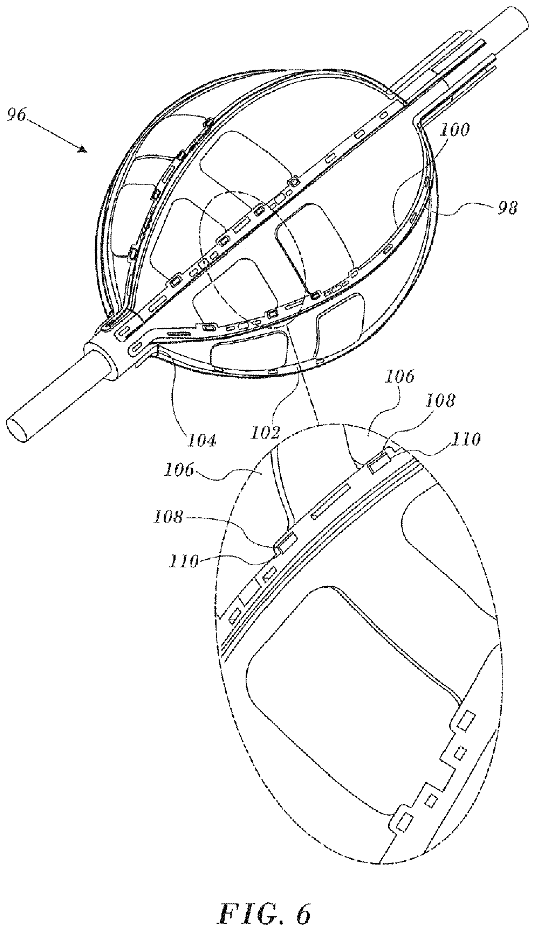

Reference is now made to Fig. 6, which is a partially schematic diagram of the distal portion of an expanded cardiac balloon catheter 96 in accordance with an alternate embodiment of the invention. A representative electromagnetic loop comprising conductors shared by ablation electrodes and thermistors is represented by a line 98 extending along and bridging splines 100, 102 across bridge connector 104. In this embodiment, as shown in the balloon at the lower right of Fig. 6, ablation electrodes 106 are printed on the surface of the balloon. Additional ablation electrodes 108, indicated by rectangles are printed atop thermistors 110. Flexible circuitry is used in the construction of the balloon catheter 96.

It will be appreciated by persons skilled in the art that the present invention is not limited to what has been particularly shown and described hereinabove. Rather, the scope of the present invention includes both combinations and sub-combinations of the various features described hereinabove, as well as variations and modifications thereof that are not in the prior art, which would occur to persons skilled in the art upon reading the foregoing description.

### ASPECTS OF THE INVENTION

1. A method, comprising the steps of:
   inserting into a body of a subject a catheter having an elongated shaft and a distal portion, the distal portion comprising an inflatable balloon assembly;
   a plurality of ablation electrodes on a surface of the balloon assembly;
   connecting the ablation electrodes to conductors extending through the shaft to supply the ablation electrodes with electrical power and connectable to a power source;
   disposing a plurality of thermistors on the balloon assembly in electrical connection with the conductors to form a resistive network; and
   obtaining electrical measurements at a plurality of access points on the conductors; and
   analyzing the electrical measurements to derive respective resistances of the thermistors.
2. The method according to aspect 1, wherein the network has nodes connected to respective voltage or current sources.
3. The method according to aspect 2, wherein the respective voltage or current sources have respective frequencies.
4. The method according to aspect 1, wherein the balloon assembly has a plurality of splines, and wherein the ablation electrodes and the thermistors are electrically connected with the splines.
5. The method according to aspect 4, wherein the splines comprise flexible circuitry, and the thermistors are mounted on the splines.
6. The method according to aspect 5, wherein there are eight access points and the splines each have four thermistors mounted thereon.
7. The method according to aspect 4, wherein the splines are connected by bridging connectors to define electromagnetic loops.
8. The method according to aspect 4, wherein there are four splines sharing a common distal electrical connection.
9. The method according to aspect 1, wherein the ablation electrodes are placed on the surface of the balloon assembly by printing.
10. The method according to aspect 1, wherein the ablation electrodes are placed on the surface of the balloon assembly by stamping.

## Claims

1. A medical apparatus, comprising:
a catheter having an elongated shaft and a distal portion, the distal portion comprising an inflatable balloon assembly;
a plurality of ablation electrodes on a surface of the balloon assembly;
conductors extending through the shaft to supply the ablation electrodes with electrical power and connectable to a power source;
a plurality of thermistors on the balloon assembly that are connected to the conductors to form a resistive network; and
a plurality of access points on the conductors for obtaining electrical measurements therefrom.

2. The apparatus according to claim 1, wherein the network has nodes connected to respective voltage or current sources.

3. The apparatus according to claim 2, wherein the respective voltage or current sources have respective frequencies.

4. The apparatus according to claim 1, wherein the balloon assembly has a plurality of splines, wherein the ablation electrodes and the thermistors are electrically connected with the splines.

5. The apparatus according to claim 4, wherein the splines comprise flexible circuitry, and the thermistors are mounted on the splines.

6. The apparatus according to claim 5, wherein there are eight access points and the splines each have four thermistors mounted thereon.

7. The apparatus according to claim 4, wherein the splines are connected by bridging connectors to define electromagnetic loops.

8. The apparatus according to claim 4, wherein there are four splines sharing a common distal electrical connection.

9. The apparatus according to claim 1, wherein the ablation electrodes are placed on the surface of the balloon assembly by printing.

10. The apparatus according to claim 1, wherein the ablation electrodes are placed on the surface of the balloon assembly by stamping.
